# FASCICULE DE BREVET EUROPEEN

(11) **EP 1 723 979 B1**
(45) Date de publication et mention de la délivrance du brevet: **30.07.2008**
(21) Numéro de dépôt: 05352019.3
(22) Date de dépôt: 23.12.2005
(51) Int. Cl.: A61M 5/31, A61M 5/315, A61M 5/28

(54) **Seringue pourvue d'une membrane protectrice**
Spritze mit einer Schutzmembran
Syringe equipped with a protective membrane

(30) Priorité: 07.01.2005 FR 0500195
(43) Date de publication de la demande: 22.11.2006
(73) Titulaire: Ranguin, Sophrone, 66240 St. Esteve (FR)
(72) Inventeur: Ranguin, Sophrone, 66240 St. Esteve (FR)

(56) Documents cités:
- WO-A-99/45851
- DE-A1- 19 731 878
- US-A- 5 466 219
- US-A1- 2001 018 575
- US-A1- 2003 065 291

## Description

Cette seringue de très faible coût reste dans l'esprit du produit à usage unique, à portée de tous. Le respect de l'asepsie rigoureuse, notions fondamentales pour combattre les maladies infectieuses et la contamination nosocomiale que tout patient et praticien doivent exiger lors de l'utilisation d'une seringue.

La présente invention concerne un dispositif pour protéger contre les fautes d'asepsie d'une seringue de ponction et d'injection de produits médicamenteux. Les seringues dites à usage unique utilisées de façon courante, pour toutes les injections et de ponctions font souvent l'objet de réutilisations multiples sans que même l'utilisateur s'en rende compte.

Par exemple lorsqu'un produit injectable se présente sous forme de solvant et de principe actif séparément, la même seringue aspire le solvant, puis l'injecte dans le principe actif, aspiré à nouveau avant d'être injecté au patient. Le plongeur saisi la plupart du temps à pleine main franchit plusieurs fois la chambre stérile. C'est déjà une très grosse faute d'asepsie et la notion d'usage unique a perdu tout son sens. Dans les services de réanimation on utilise très souvent des moteurs dits auto-pousseurs pour actionner les seringues.

Les pistons et plongeurs sont exposés des heures entières à la poussière et représentent un bouillon de culture véritable source d'infections nosocomiales.

Les dessins annexes illustrent l'invention :

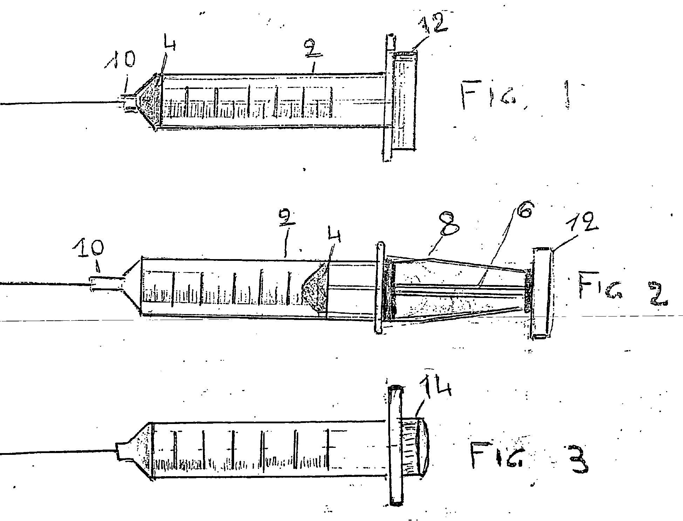

• La figure 1 représente la seringue en position fermée.
• La figure 2 représente la seringue ouverte.
• La figure 3 représente la seringue mettant en évidence son support de fixation d'une extrémité de la membrane réalisé dans son moulage initial d'un prolongement de taille en fonction du volume de la seringue en forme d'épaulement.

Les éléments constitutifs de la seringue d'injection et de ponction se composent d'un corps de seringue cylindrique (2) servant de réservoir de fluide. L'extrémité distale du dit corps de seringue est formée d'un embout perméable (10) destiné d'une part à l'écoulement et d'autre part à recevoir les instruments de ponction et d'injection adaptables : aiguilles, prolongateurs et autres cathéters indispensables à l'écoulement du fluide vers le patient ou vers le réservoir cylindrique (2). Le piston (4) solidement inséré à l'extrémité distale du plongeur (6) dans son mouvement de va et vient à l'intérieur du réservoir cylindrique (2) assure par son étanchéité parfaite la circulation du fluide aspiré ou injecté, le plongeur (6) destiné à actionner le piston (4) coulissant dans le réservoir cylindrique (2) travaillant en gavage et en extraction contraint impérativement le plongeur (6) à s'éloigner du réservoir cylindrique ou corps de seringue (2).

Une membrane tubulaire (8) en latex souple dans son rôle protecteur et isolant est fixée d'une part sur l'extrémité proximale externe du réservoir cylindrique (2) et d'autre part sur l'extrémité proximale du plongeur (6) qui se trouve ainsi protégé de tout contact d'objets septiques lors de son déplacement extérieur du réservoir cylindrique; le déplissement aisé du tube en latex (8) est rendu facile car la prise en main sur la partie proximale du plongeur s'effectue uniquement sur son support rigide (12) du dit plongeur (6). La taille de ce support (12) de prise en main du plongeur (6) évolue en fonction de la capacité du réservoir cylindrique (2). A noter que le plongeur (6) dans sa course extérieure maximale conduit le piston (4) à l'extrémité proximale du réservoir cylindrique (2).

La membrane tubulaire latex (8) en se déplissant sous forme d'habillage recouvre totalement cette partie du plongeur (6) très vulnérable caractérisée en ce que la seringue (2) comporte à son sommet, dans un moulage initial un épaulement (14) permettant d'insérer une extrémité de la membrane en latex (8), l'autre extrémité de cette membrane en latex (8) venant s'insérer au sommet du plongeur (6) solidarisant ainsi le couple seringue (2) et plongeur (6).

Grâce à cette barrière étanche l'asepsie rigoureuse est respectée, aucune transmission de germe n'est possible. Lorsque le piston (4) est refoulé en gavage jusqu'à l'extrémité distale du réservoir cylindrique (2), les plicatures de la membranes en latex (8) assemblées au sommet du réservoir cylindrique (2) à son extrémité proximale ne sont ni visibles, ni palpables puisque la partie (12) de prise en main du plongeur (6), de taille en fonction de la capacité du réservoir cylindrique (2) recouvre et protège la membrane en latex (8). Ce support rigide (12) évite toutes destructions possibles au cours des diverses manoeuvres, emballage, stérilisation, transport, ongles et tous objets traumatisants. Ceci remédie au problème de déchirure ou rupture que l'on pourrait avoir avec une seringue telle que celle décrite dans EP 253949.

## Revendications

1. Dispositif pour protéger contre les fautes d'asepsie d'une seringue de ponction et d'injection de produits médicamenteux comprenant une membrane tubulaire en latex (8), insérée à la fois à l'extrémité proximale du réservoir cylindrique de la seringue (2) et à l'extrémité proximale du plongeur (6) **caractérisé en ce que** le plongeur (6) comprend une partie de prise en main (12) qui recouvre et protège la membrane en latex (8) de telle façon que lorsque le piston (4) est refoulé en gavage jusqu'à l'extrémité distale du réservoir cylindrique (2), les plicatures de la membrane en latex (8) ne sont ni visibles, ni palpables

2. Dispositif selon la revendication 1 **caractérisé en ce que** la membrane tubulaire en latex (8) comporte à chaque extrémité un renfort sous forme d'anneau permettant à la membrane en latex (8) d'être solidement insérée à la fois à l'éxtrémité proximale du reservoir cylindrique de la seringue (2) et à l'éxtrémité proximale du plongeur (6).

## Claims

1. A Device, to protect against asepsis problems caused by using a syringe and the injection of medicinal products, being made up of a latex tubular membrane (8), inserted at the proximal end of the cylinder tank of the syringe (2) and at the proximal end of the plunger (6). The plunger (6) being made up of the part of the syringe taken in the hand (12) which is covered and protected by a latex membrane (8). When the piston (4) is repressed to the distal end of the cylinder tank (2) the foldings of the latex membrane (8) are neither visible nor palpable.

2. This Device claims are **characterised by** a latex tubular membrane, included at each end a ring allowing the latex membrane (8) to be solidly inserted at the proximal end of the cylinder tank of the syringe (2) and the proximal end of the plunger (6).

## Patentansprüche

1. Anlage, um gegen die Fehler der Keimfreiheit einer Spritze der Punktion und Einspritzung heilkräftiger Produkte zu schützen, die eine röhrenförmige Membran in Latex (8) enthalten, die zugleich am proximale Ende des zylindrischen Reservoirs der Spritze (2) und am proximale Ende des Tauchers (6) hineingelegt ist, der darin charakterisiert ist, dass der Taucher (6), einen Teil Handlicher Einsatz (12) versteht, der einzieht und die Membran in Latex (8), schützt, dass, wenn der Kolben (4) zum Ende zurückgedrängt wird, bis zu distale Extremität des zylindrischen Reservoirs (2), der Plicatures der Membran in Latex (8) sind weder sichtbar, noch tastbar.

2. Anlage nach der Forderung (1) Charakterisierter darin, was die röhrenförmige Membran in Latex (8) an jedem Ende in Form von Ring enthält, der der Membran in Latex (8) ermöglicht, am proximale Ende des zylindrischen Reservoirs der Spritze (2) und des Tauchers (6) am proximale Ende fest hineingelegt zu werden.
